# EUROPÄISCHE PATENTSCHRIFT

(11) **EP 1 637 097 B1**
(45) Veröffentlichungstag und Bekanntmachung des Hinweises auf die Patenterteilung: **03.02.2010**
(21) Anmeldenummer: 05019266.5
(22) Anmeldetag: 05.09.2005
(51) Int. Cl.: A61F 2/76

(54) **Klemmfreilauf und seine Verwendung in Prothesen**
One way clutch and its use in prostheses
Embrayage unidirectionnelle et son utilisation dans les prothèses

(30) Priorität: 08.09.2004 DE 102004043805
(43) Veröffentlichungstag der Anmeldung: 22.03.2006
(73) Patentinhaber: Otto Bock HealthCare GmbH, 37115 Duderstadt (DE)
(72) Erfinder: Mosler, Lüder, 37115 Duderstadt (DE); Hillman, Martin, 37115 Duderstadt (DE)
(74) Vertreter: Lins, Edgar

(56) Entgegenhaltungen:
- WO-A-99/04178
- WO-A1-2006/076011
- DE-C- 605 879
- DE-C- 874 684
- US-A1- 2004 030 410
- US-B1- 6 267 787
- "Handbook of Stock Drive products/Sterling Instruments Components" [Online] 1998, STOCK DRIVE PRODUCTS/STERLING INSTRUMENTS , JERICHO TUMPIKE NY USA , XP002364454 Gefunden im Internet: URL:http://www.sdp-si.com/D785/HTML2/D785B RG062_1.html> [gefunden am 2006-01-25] Seite 5-62 - Seite 5-64 * das ganze Dokument *

## Beschreibung

Die Erfindung betrifft einen Klemmfreilauf mit einer eine Welle umschließenden Hülse, in der die Welle axial verschiebbar gelagert ist, mit Rollkörpern, die in zwischen Welle und Hülse derart geformten Kammern so angeordnet sind, dass sie für eine relative Drehung zwischen Welle und Hülse in einer ersten Richtung rollen, während ein in entgegen gesetzter Richtung wirkendes Drehmoment zur Klemmung der Rollkörper in ihrer Kammer führt, mit einem an der Welle angeordneten Übertragungselement und mit einem Betätigungselement zum axialen Verschieben des an der Welle angeordneten Übertragungselements, wobei die Welle aus einem Hohlwellenstück und einem darin axial verschiebbar, aber verdrehsicher gelagerten Innenstück besteht und dass das Betätigungselement und das Übertragungselement am Innenstück angeordnet sind.

Derartige Klemmfreiläufe sind als Standard-Bauelemente bekannt und bewirken einen möglichst geringen Widerstand für eine Drehung der Welle in einer Richtung und eine möglichst sofortige Klemmung, falls mit dem Übertragungselement ein Drehmoment in der entgegen gesetzten Richtung ausgeübt wird, wodurch eine relative Bewegung zwischen der Welle und der Hülse verhindert wird.

Durch DE 605 879 ist ein derartiger Klemmfreilauf als Freilaufkupplung für Kraftfahrzeuge und durch AT 125 797 als Schaltkupplung für ein Geschwindigkeitswechselgetriebe bekannt.

Durch DE 874 684 ist ferner eine ein- und ausrückbare Wellenkupplung bekannt, bei die Kraftübertragung über Kugeln erfolgt, die an schrägen Laufflächen der treibenden und der getriebenen Wellen anliegen können, wobei eine axiale Verschiebung der Wellen zueinander zu einem das Drehmoment übertragenden Klemmen der Kugeln führt. Eine ähnliche Lösung, bei der die Mitnahmewirkung von Kugeln durch eine axiale Verschiebung mit konischen Laufflächen erzielt wird, ist aus JP 2004 125 140 A bekannt.

Durch US 3,279,571 ist eine Kupplungsanordnung bekannt, bei der eine Antriebswelle drehfest mit einem axial sich konisch verjüngenden Zylinder verbunden ist, dessen Mantelflächen Laufflächen von Kugeln bilden. Durch eine axiale Bewegung der Wellen zueinander werden die Kugeln den Kammern der angetriebenen Welle nach außen gedrückt und gelangen in Eingriff mit einer Kammerwandung, die so ausgebildet ist, dass eine Mitnahme in eine Richtung und ein Freilauf in der anderen Richtung erfolgen. Dabei kann sich die Wandung der Kammer in der mitgenommenen Welle verjüngen, um so eine Klemmwirkung mit einer in der Kammer laufenden Kugel in einer Drehrichtung zu bewirken, während in der anderen Drehrichtung die Kugel freiläuft. Durch eine axiale Drehung kann mit dem konischen Zylinder des antreibenden Rades eine Mitnahmefunktion der Kugeln ausgeschaltet werden.

Durch WO 99/04 178 ist ferner bekannt, für eine Freilauf-Ratscheneinrichtung zylindrische Rollen als Übertragungselement zu verwenden.

Ein Anwendungsgebiet eines derartigen Klemmfreilaufs ist seine Verwendung in einer Rasteinheit (Shuttle Lock) bei Prothesen. Als bettende Lage zwischen einem Prothesenschaft und dem amputierten Stumpf werden zunehmend Stumpfüberzüge aus einem weichen Elastomer, so genannte Liner, eingesetzt. Zur Fixierung des Liners am Prothesenschaft sind diese am distalen Ende mit einer festen Kappe mit eingeschraubtem Stift versehen. Der Stift weist umlaufende Nuten auf, die mit einem Zahnrad als Übertragungselement des Klemmfreilaufs zusammenwirken, wobei die Welle des Klemmfreilaufs senkrecht zu der Längsachse des Stiftes liegt. Das Ende des Liners kann somit nur distal verschoben werden, während eine Rückbewegung durch den Klemmfreilauf verhindert wird. Zum Lösen der Verriegelung wird die Freilaufwelle axial verschoben, wodurch das Zahnrad axial ausgerückt wird und außer Eingriff mit dem Stift gelangt, sodass der Stift zurückgezogen werden kann. Die bekannten Klemmfreiläufe dieser Art sind mit Nadelrollen aufgebaut, die sich auf kleinem Raum gut realisieren lassen. In der Praxis zeigt es sich jedoch, dass im arretierten Zustand der Klemmfreilauf erhebliche Klemmkräfte erzeugt, die die axiale Verschiebung der Welle im Freilauf behindern. Die für die axiale Verschiebung am Betätigungselement aufzubringenden Kräfte können daher so groß werden, dass ein benötigtes Lösen der Verriegelung durch den Patienten nicht erfolgen kann. Die Ursache hierfür kann eine ungenügende Passform des Schaftes sein, wodurch sich der distale Linerteller im Prothesenschaft verspannen kann. Im Sitzen kann durch die Hebelwirkung des Stumpfes die Auslösekraft zum Lösen der Verriegelung nochmals deutlich ansteigen.

Ein Klemmfreilauf der eingangs erwähnten Art ist durch US 6,267,787 B1 bekannt. Dabei ist die Welle der Klemmkupplung zweiteilig ausgeführt, sodass eine Trennung der Funktionen "axiales Verschieben" und "Klemmung" erfolgt. Die eigentliche Freilaufunktion wird an der Außenseite des Hohlwellenstücks in Verbindung mit der Hülse realisiert, während die axiale Verschiebung an dem Innenstück relativ zu dem Hohlwellenstück vorgenommen wird. Bei der Verwendung des Klemmfreilaufs für die Arretierung eines Prothetikteils wird dadurch erreicht, dass die Entarretierung durch den Patienten mit im wesentlich gleich bleibenden Entarretierungskräften erfolgen kann. Die Verdrehsicherung zwischen dem Innenstück und dem Hohlwellenstück wrid dadurch erreicht, dass ein Stift senkrecht zur Achse in miteinander fluchtenden Bohrungen des Hohlwellenstücks und des Innenstücks passig gelagert ist.

Gemäß der vorliegenden Erfindung wird die Verdrehsicherung dadurch erreicht, dass das Innenstück einen mehreckigen Außenumfang und das Hohlwellenstück ein zum Außenprofil des Innenstücks komplementäres Innenprofil aufweist. Hierdurch lässt sich eine einfachere und leichter herstellbare Ausbildung des Klemmfreilaufs erreichen. In einer bevorzugten Ausführungsform weist das Innenstück ein viereckiges Außenprofil und das Hohlwellenstück ein Innenprofil in Form eines Vierecks auf.

In einer weiteren bevorzugten Ausführungsform ist das Innenstück für die axiale Verschiebung an dem Hohlwellenstück mittels Kugeln gelagert. Die Kugellagerung kann zur Erzielung der Verdrehsicherung ausgenutzt werden, indem wenigstens eine Kugel in wenigstens einer Linearführung angeordnet ist.

Die bevorzugten Anwendung des erfindungsgemäßen Klemmfreilaufs erfolgt zur Verriegelung eines linear bewegbaren Prothetikteils, dessen Bewegung mittels eines mit dem Übertragungselement des Klemmfreilaufs zusammenwirkenden Koppelelements auf den Klemmfreilauf übertragbar ist, wobei durch die axiale Verschiebung des Innenstücks der Welle das Übertragungselement und das Koppelelement außer Eingriff bringbar sind.

Die Erfindung soll im Folgenden anhand eines in der Zeichnung dargestellten Ausführungsbeispiels näher erläutert werden. Es zeigen:
- Figur 1: eine explodierte Darstellung eines erfindungsgemäßen Klemmfreilaufs
- Figur 2: eine Schnittdarstellung des zusammengebauten Klemmfreilaufs gemäß Figur 1
- Figur 3: einen schematischen Hochschnitt durch eine Rasteinheit für ein Prothe- tikteil mit einem Klemmfreilauf gemäß Figur 1
- Figur 4: einen Hochschnitt in einer gegenüber Figur 3 um 90° gedrehten Ebene.

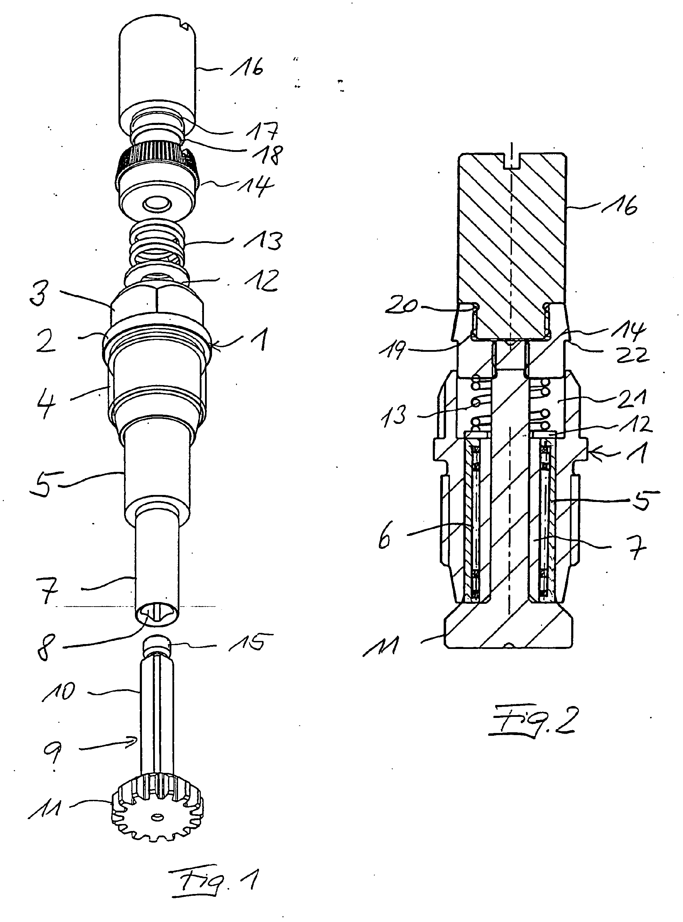

Der in den Figuren 1 und 2 dargestellte Klemmfreilauf weist ein hohlzylindrisches Gehäuse 1 auf, das auf einer Seite einer Ringschulter 2 als Sechskant 3 ausgeformt ist und auf der anderen Seite ein Außengewinde 4 aufweist. In das Gehäuse 1 wird eine Hülse 5 eingesetzt, die auf ihrer Innenwandung Kammern aufweist, in denen Nadelzylinder 6 (Figur 2) als Rollkörper eines an sich bekannten Freilaufs gelagert sind. Die Hülse 5 bildet den Freilauf zusammen mit der Außenwandung eines Hohlwellenstücks 7, das in die Buchse passig einsetzbar ist. Das Hohlwellenstück 7 weist ein Innenprofil 8 in Form eines Vierecks auf, in das ein Innenstück 9 mit einem entsprechenden viereckigen Außenprofil 10 drehfest eingesetzt wird. Die Einschubbewegung des Innenstücks 9 in das Innenprofil 8 des Hohlwellenstücks 7 wird durch ein am Ende des Innenstücks 9 angeordnetes Zahnrad 11 begrenzt, dessen Außendurchmesser der Zähne und Innendurchmesser der Nutgründe größer als der Außendurchmesser des Hohlwellenstücks 7 ist.

Das andere Ende des Gehäuses 1 ist mit einer ringförmigen Anschlagscheibe 12 einer als Druckfeder 13 eingesetzten Spiralfeder und einem in das Gehäuse 1 drehbar einsetzbaren Kopfstück 14 bestückt. Das Kopfstück 14 ist durch Presssitz, eine Schnappverbindung oder eine Verschraubung mit einem Ansatzstück 15 des Innenstücks 9 drehfest verbunden. In das Kopfstück 14 ist ein Drehknopf 16 als Betätigungselement mittels einer Rastnut 17 und einem Rastwulst 18 einschnappbar, wobei das Kopfstück 14 eine entsprechend komplementär ausgebildete Rastnut 19 und einen Rastwulst 20 aufweist.

Wie Figur 2 verdeutlicht, ist in dem Gehäuse 1 eine ringförmige, axial erstreckte Kammer 21 oberhalb der in das Gehäuse 1 eingepressten Hülse 5 vorgesehen, in der die Druckfeder 13 sowie das Kopfstück 14 aufgenommen wird. Das Kopfstück 14 ist gegen die Rückstellkraft der Druckfeder 13 in das Innere des Gehäuses axial verschiebbar, bis ein umlaufend vorstehender Rand 22 des Kopfstücks 14 auf den Rand des Gehäuses 1 auftrifft und somit die Einschubbewegung begrenzt.

Die Einschubbewegung des Kopfstücks 14, ausgelöst am Betätigungselement 16, führt zu einer entsprechenden axialen Verschiebung des als Übertragungselement dienenden Zahnrades 11, um dieses außer Eingriff von einem zugehörigen Kopplungselement, beispielsweise einem in der Darstellung der Figur 2 senkrecht zur Zeichenebene mit seiner Längsachse ausgerichteten und mit entsprechenden Nuten versehenen, longitudinal verschiebbaren Stift zu bringen.

Es ist erkennbar, dass die axiale Verschiebung des Zahnrades 11 durch eine Verschiebung des Innenstücks 9 relativ zum Hohlwellenstück 7 erfolgt, während die Klemmwirkung durch die im dargestellten Ausführungsbeispiel als Nadelrollen ausgebildeten Rollkörper zwischen der Außenwandung des Hohlwellenstücks 7 und der Innenwandung der Hülse 5 erfolgt, sodass die Axialverschiebung von der Klemmung der Nadelrollen 6 unabhängig ist.

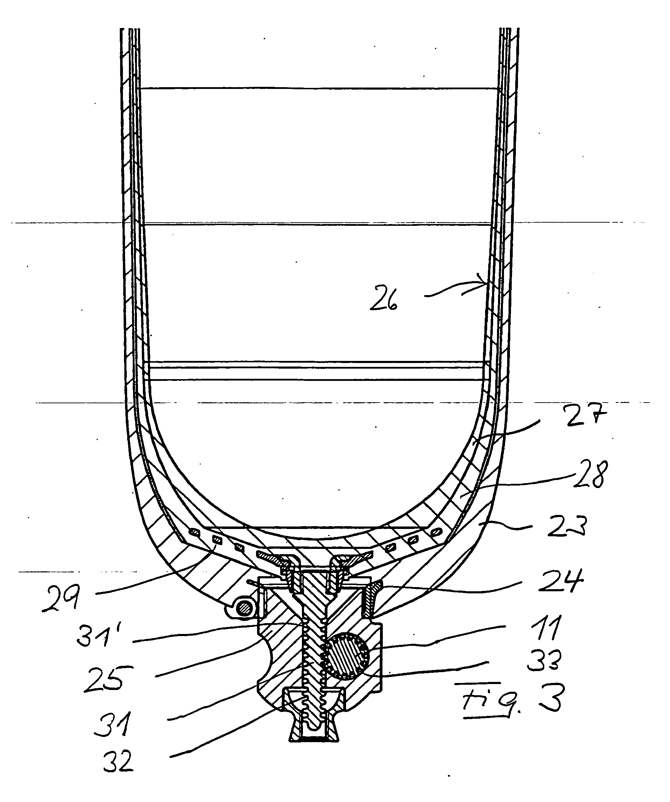

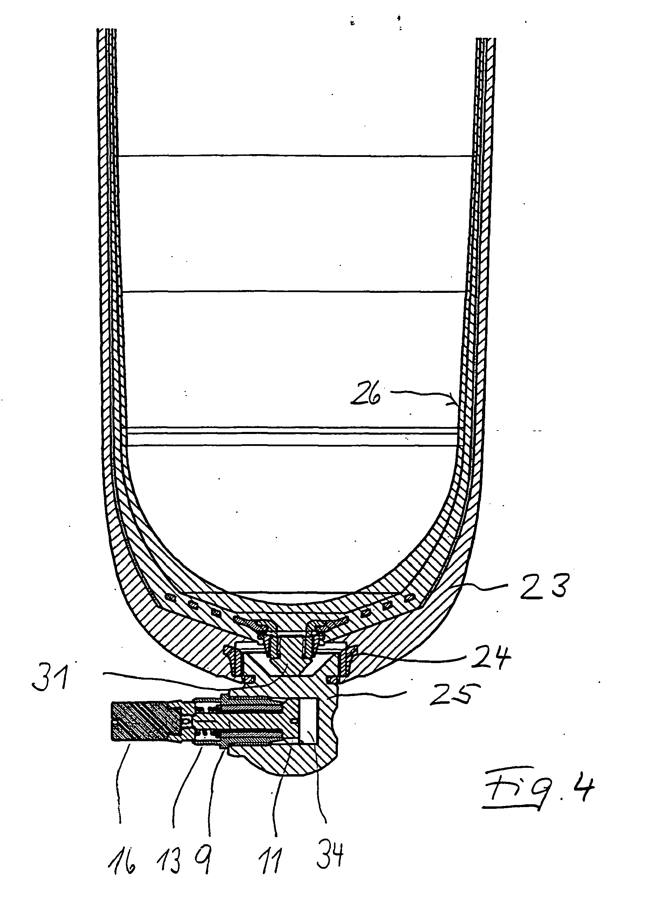

Die Figuren 3 und 4 zeigen einen in üblicher Weise trichterförmig ausgebildeten Prothesenschaft 23 mit einem topfartig geschlossenen distalen Ende, in das ein Schaftadapter 24 eingesetzt ist, der zur Befestigung eines Rastgehäuses 25 dient. Die Verbindung eines Amputationsstumpfes mit dem Prothesenschaft 23 erfolgt über einen über den Amputationsstumpf gezogenen Liner 26 aus einem weichen, hautfreundlichen Innenmaterial 27 und einem vorzugsweise mit einer gleitfähigen Oberfläche ausgebildeten Außenmaterial 28, das im Bereich des geschlossenen distalen Endes mit einer Gewebeverstärkung 29 versehen ist. Zentrisch weist der Liner 26 am distalen Ende eine Schraubhülse 30 auf, in die ein Arretierungsstift 31 mit einem Außengewinde eingeschraubt ist. Der Arretierungsstift ist als länglicher Bolzen ausgebildet, der mit radial umlaufenden, zueinander parallelen Noten 32 versehen ist. Der Arretierungsstift 31 ist in einer zentrischen Führungsbohrung 31 des Rastgehäuses 25 in Achsrichtung verschiebbar geführt. Das Rastgehäuse 25 weist ferner eine horizontale Bohrung 33 auf, in die der in den Figuren 1 und 2 beschriebene Klemmfreilauf so eingesetzt ist, dass das Zahnrad 11 im Betriebszustand in die Nuten 32 des Arretierungsstifts 31 eingreift, wie dies in Figur 3 dargestellt ist. Durch die Wirkung des Klemmfreilaufs wird erreicht, dass der Arretierungsstift 31 durch Druck auf den Liner 26 in distale Richtung verschiebbar ist, wohingegen ein Zurückziehen in proximaler Richtung durch den Klemmfreilauf verhindert wird.

Figur 4 zeigt einen Schnitt entlang einer Hochebene, die gegenüber der Schnittebene der Figur 3 um 90° gedreht und im Bereich des Klemmfreilaufs seitlich verschwenkt ist, sodass der Schnitt mittig durch die horizontale Bohrung 33 und den Klemmfreilauf verläuft. Es ist erkennbar, dass das Innenstück 9 des Klemmfreilaufs mit dem endseitigen Zahnrad 11 in einer Kammer 34 gelagert ist, die ein Eindrücken des Innenstücks 9 mittels des Drehknopfs 16 soweit ermöglicht, dass dadurch das Zahnrad 11 außer Eingriff der Nuten 32 des Arretierungsstifts 31 gelangt, sodass durch das Eindrücken des Drehknopfs 16 der Arretierungsstift 31 freigegeben und somit nach oben aus seiner Führungsbohrung 31 in dem Rastgehäuse 25 herausgezogen werden kann. Aufgrund der beschriebenen erfindungsgemäßen Konstruktion ist die Entarretierung unabhängig von den durch die Nadelzylinder 6 aufgebrachten Klemmkräfte und daher auch im geklemmten Zustand unproblematisch möglich.

## Patentansprüche

1. Klemmfreilauf mit einer eine Welle (7, 9) umschließenden Hülse (5), in der die Welle (7, 9) axial verschiebbar gelagert ist, mit Rollkörpern (6), die in zwischen Welle (7, 9) und Hülse (5) derart geformten Kammern so angeordnet sind, dass sie für eine relative Drehung zwischen Welle (7, 9) und Hülse (5) in einer ersten Richtung rollen, während ein in entgegen gesetzter Richtung wirkendes Drehmoment zur Klemmung der Rollkörper (6) in ihrer Kammer führt, mit einem an der Welle (7, 9) angeordneten Übertragungselement (11) und mit einem Betätigungselement (16) zum axialen Verschieben des an der Welle (7, 9) angeordneten Übertragungselements (11), wobei die Welle (7, 9) aus einem Hohlwellenstück (7) und einem darin axial verschiebbar, aber verdrehsicher gelagerten Innenstück (9) besteht und dass das Betätigungselement (16) und das Übertragungselement (11) am Innenstück (9) angeordnet sind, **dadurch gekennzeichnet, dass** das Innenstück (9) einen mehreckigen Außenumfang und das Hohlwellenstück (7) einen komplementäres Innenprofil (8) aufweist

2. Klemmfreilauf nach Anspruch 1, **dadurch gekennzeichnet, dass** die axiale Verschiebung des Innenstück (9) gegen die Rückstellkraft einer Feder (13) erfolgt.

3. Klemmfreilauf nach Anspruch 1 oder 2, **dadurch gekennzeichnet, dass** zur axialen Verschiebung das Innenstück (9) an dem Hohlwellenstück (7) mittels Kugeln gelagert ist.

4. Klemmfreilauf nach Anspruch 3, **dadurch gekennzeichnet, dass** wenigstens eine Kugel in wenigstens einer Linearführung angeordnet ist.

5. Klemmfreilauf nach einem der Ansprüche 1 bis 4, **dadurch gekennzeichnet, dass** das Übertragungselement ein Zahnrad (11) ist.

6. Verwendung des Klemmfreilaufs nach einem der Ansprüche 1 bis 5 zur Verriegelung eines linear bewegbaren Prothetikteils, dessen Bewegung mittels eines mit dem Übertragungselement (11) des Klemmfreilaufs zusammenwirkenden Koppelelements auf den Klemmfreilauf übertragbar ist, wobei durch die axiale Verschiebung des Innenstücks (9) der Welle (7, 9) das Übertragungselement (11) und das Koppelelement außer Eingriff bringbar sind.

## Claims

1. Clamping freewheel having a sleeve (5) which encloses a shaft (7, 9) and in which the shaft (7, 9) is mounted in an axially displaceable manner, having rolling elements (6) which are arranged in chambers, formed between shaft (7, 9) and sleeve (5), in such a way that they roll in a first direction for a relative rotation between shaft (7, 9) and sleeve (5), whereas a torque acting in the opposite direction leads to the clamping of the rolling elements (6) in their chamber, having a transmission element (11) arranged on the shaft (7, 9), and having an actuating element (16) for the axial displacement of the transmission element (11) arranged on the shaft (7, 9), wherein the shaft (7, 9) consists of a hollow shaft piece (7) and an inner piece (9) axially displaceable therein, but mounted in a rotationally locked manner, and wherein the actuating element (16) and the transmission element (11) are arranged on the inner piece (9), **characterized in that** the inner piece (9) has a polygonal outer circumference and the hollow shaft piece (7) has a complementary inner profile (8).

2. Clamping freewheel according to Claim 1, **characterized in that** the axial displacement of the inner piece (9) is effected against the restoring force of a spring (13).

3. Clamping freewheel according to one of Claims 1 or 2, **characterized in that**, for the axial displacement, the inner piece (9) is mounted on the hollow shaft piece (7) by means of balls.

4. Clamping freewheel according to Claim 4, **characterized in that** at least one ball is arranged in at least one linear guide.

5. Clamping freewheel according to one of Claims 1 to 4, **characterized in that** the transmission element is a gear (11).

6. Use of the clamping freewheel according to one of Claims 1 to 5 for locking a linearly movable prosthetic part, the movement of which can be transmitted to the clamping freewheel by means of a coupling element interacting with the transmission element (11) of the freewheel, it being possible for the transmission element (11) and the coupling element to be disengaged by the axial displacement of the inner piece (9) of the shaft (7, 9).

## Revendications

1. Embrayage unidirectionnel comprenant : un manchon (5) entourant un arbre (7, 9) et dans lequel l'arbre (7, 9) est monté axialement déplaçable ; des corps roulants (6) qui sont agencés dans des chambres ainsi formées entre arbre (7, 9) et manchon (5) qu'ils roulent pour une rotation relative entre arbre (7, 9) et manchon (5) dans une première direction, tandis qu'un moment agissant dans la direction opposée conduit au blocage des corps roulants (6) dans leur chambre ; un élément de transmission (11) agencé sur l'arbre (7, 9) ; et un élément d'actionnement (16) pour le déplacement axial de l'élément de transmission (11) agencé sur l'arbre (7, 9) ; et dans lequel l'arbre (7, 9) consiste en une pièce d'arbre creux (7) et une pièce interne (9) qui y est montée axialement déplaçable mais sans possibilité de rotation, l'élément d'actionnement (16) et l'élément de transmission (11) étant agencés sur la pièce interne (9), **caractérisé en ce que** la pièce interne (9) présente un périmètre extérieur à plusieurs angles et la pièce d'arbre creux (7) présente un profil interne (8) complémentaire.

2. Embrayage unidirectionnel selon la revendication 1, **caractérisé en ce que** le déplacement axial de la pièce interne (9) s'effectue contre la force de rappel d'un ressort (13).

3. Embrayage unidirectionnel selon la revendication 1 ou 2, **caractérisé en ce que** pour le déplacement axial, la pièce interne (9) est montée sur la pièce d'arbre creux (7) au moyen de billes.

4. Embrayage unidirectionnel selon la revendication 3, **caractérisé en ce qu'**au moins une bille est agencée dans au moins un guidage linéaire.

5. Embrayage unidirectionnel selon l'une des revendications 1 à 4, **caractérisé en ce que** l'élément de transmission est une roue dentée (11).

6. Utilisation de l'embrayage unidirectionnel selon l'une des revendications 1 à 5 pour verrouiller une pièce prothétique linéairement mobile, dont le mouvement est transmis à l'embrayage unidirectionnel au moyen d'un élément de couplage coopérant avec l'élément de transmission (11) de l'embrayage unidirectionnel, l'élément de transmission (11) et l'élément de couplage pouvant être mis hors de prise grâce au déplacement axial de la pièce interne (9) de l'arbre (7, 9).
